(19) Europäisches Patentamt

European Patent Office

Office européen des brevets

(11) Veröffentlichungsnummer: **0 604 717 A2**

# (12) EUROPÄISCHE PATENTANMELDUNG

(21) Anmeldenummer: **93116199.6**

(22) Anmeldetag: **07.10.93**

(51) Int. Cl.5: **A61K 7/09**

(30) Priorität: **02.12.92 DE 4240471**

(43) Veröffentlichungstag der Anmeldung:
**06.07.94 Patentblatt 94/27**

(84) Benannte Vertragsstaaten:
**DE ES FR GB IT**

(71) Anmelder: **Wella Aktiengesellschaft**
**Berliner Allee 65**
**D-64295 Darmstadt(DE)**

(72) Erfinder: **Mager, Herbert, Dr.**
**21, Route du Roule**
**CH-1723 Marly(CH)**
Erfinder: **Clausen, Thomas, Dr.**
**Ernst-Pasqué-Strasse 35 A**
**D-64665 Alsbach(DE)**
Erfinder: **Hoch, Dietrich**
**Riedstrasse 30**
**D-64319 Pfungstadt-Eich(DE)**

(54) Verfahren zur dauerhaften Haarverformung und Mittel zur Durchführung dieses Verfahrens.

㊐ Gegenstand der Erfindung ist ein Verfahren zur dauerhaften Haarverformung, bei dem man das Haar bevor und/oder nachdem man es in die gewünschte Form bringt mit einem Haarverformungsmittel auf der Basis eines haarkeratinreduzierenden Wirkstoffes behandelt, mit Wasser spült, anschließend mit einem Fixiermittel auf der Basis eines oxidierenden Wirkstoffes oxidativ nachbehandelt, mit Wasser spült, zur Frisur legt und sodann trocknet, welches dadurch gekennzeichnet ist, daß das Haarverformungsmittel weniger als 2,5 Gewichtsprozent eines nichtionischen Tensides enthält und das Fixiermittel 0,01 bis 6 Gewichtsprozent eines kationischen Tensides und 0,01 bis 6 Gewichtsprozent eines kationischen Polymers enthält, sowie ein 2-Komponenten-Mittel zur Durchführung dieses Verfahrens.

EP 0 604 717 A2

Die Erfindung betrifft ein Verfahren zur dauerhaften Haarverformung sowie ein 2-Komponenten-Mittel zur Durchführung dieses Verfahrens.

Bekanntlich besteht die klassische Technik zur Durchführung der dauerhaften Haarverformung darin, daß in einer ersten Stufe die Disulfidbindungen des Haarkeratins mit Hilfe eines Mittels, welches einen reduzierenden Wirkstoff enthält, (Haarverformungsmittel) geöffnet werden, sodann das Haar in die gewünschte Form gebracht wird und anschließend die Disulfbindungen unter Verwendung eines einen oxidierenden Wirkstoff enthaltenden Mittels (Fixiermittel) wiederverknüpft werden.

Als reduzierender Wirkstoff werden vorzugsweise Mercaptane oder Sulfite verwendet, während als oxidierender Wirkstoff Wasserstoffperoxid oder Alkalibromate eingesetzt werden.

Es ist bekannt, daß durch dieses Verfahren zur dauerhaften Haarverformung die Struktur der Haare geschwächt und das Aussehen und der Griff der Haare verschlechtert wird.

Es wurden bereits mehrere Versuche gemacht, dieses Verfahren bezüglich der haarpflegenden Eigenschaften zu verbessern, indem dem Haarverformungsmittel und/oder Fixiermittel haarkonditionierend wirkende Stoffe, wie zum Beispiel kationische Tenside oder Polymere, zugesetzt wurden. Hierdurch ergaben sich jedoch eine Vielzahl von Problemen, da die Rezepturen für die Haarverformungsmittel beziehungsweise Fixiermittel immer komplizierter wurden, wodurch die Wechselwirkung der einzelnen Rohstoffe untereinander beziehungsweise mit dem Haar oder anderen Haarbehandlungsmitteln, wie zum Beispiel Haarkuren, nicht mehr voraussehbar waren. So kann es vorkommen, daß die pflegende Wirkung trotz eines hohen Gehaltes an pflegenden Wirkstoffen in dem Haarverformungsmittel und/oder Fixiermittel nicht zufriedenstellend ist. Ein weiteres Problem besteht darin, daß in bestimmten Fällen bei dem derzeit üblichen Verfahren zum einen unerklärlich hohe Konzentrationen an kationischen Polymeren erforderlich sind und zum anderen durch eine im Anschluß an die Fixierung durchgeführte Nachbehandlung mit einer kationischen Haarkur die gewünschte Verbesserung der Haarstruktur nicht erzielt wird, sondern Griff und Kämmbarkeit der Haare durch diese zusätzliche Nachbehandlung sogar verschlechtert werden (sogenannter "Antieffekt").

Aufgabe der vorliegenden Erfindung ist es daher, ein Verfahren zur dauerhaften Haarverformung zur Verfügung zu stellen, bei dem die vorstehend beschriebenen Nachteile nicht auftreten und sowohl eine gleichmäßige Verformung als auch eine Verbesserung von Griff und Kämmbarkeit der Haare erzielt wird. Weiterhin soll die Zahl der eingesetzten Wirkstoffe möglichst gering sein, um kostengünstige und überschaubare Rezepturen des Haarverformungsmittels und Fixiermittels zu ermöglichen.

Es wurde nun gefunden, daß eine Verbesserung des bisher üblichen Verfahrens durch die gemeinsame Verwendung eines speziellen Haarverformungsmittels und einer kationischen Fixierung erreicht werden kann.

Gegenstand der vorliegenden Erfindung ist daher ein Verfahren zur dauerhaften Haarverformung, bei dem man das Haar bevor und/oder nachdem man es in die gewünschte Form bringt mit einem Haarverformungsmittel auf der Basis eines haarkeratinreduzierenden Wirkstoffes behandelt, mit Wasser spült, anschließend mit einem Fixiermittel auf der Basis eines oxidierenden Wirkstoffes oxidativ nachbehandelt, mit Wasser spült, zur Frisur legt und sodann trocknet, welches dadurch gekennzeichnet ist, daß ein Haarverformungsmittel, welches weniger als 2,5 Gewichtsprozent eines nichtionischen Tensides enthält, und ein Fixiermittel, welches 0,01 bis 6 Gewichtsprozent eines kationischen Tensides und 0,01 bis 6 Gewichtsprozent eines kationischen Polymers enthält, verwendet wird.

Bei dem erfindungsgemäßen Verfahren wird das Haar gewaschen, mit einem Handtuch frottiert, gegebenenfalls mit einem Teil des Haarverformungsmittels vorgefeuchtet, in einzelne Strähnen aufgeteilt, und auf Wickler gewickelt. Der Durchmesser der Wickler beträgt hierbei, je nachdem ob eine Dauerwellung oder eine Entkräuselung der Haare gewünscht wird, entweder 5 bis 15 Millimeter oder 15 bis 35 Millimeter. Auf das gewickelte Haar wird sodann eine für die dauerhafte Haarverformung ausreichende Menge des Haarverformungsmittels aufgetragen. Die für die dauerhafte Haarverformung erforderliche Gesamtmenge des Haarverformungsmittels beträgt in der Regel 80 bis 120 Gramm.

Das verwendete Haarverformungsmittel enthält einen üblichen haarkeratinreduzierenden Wirkstoff sowie weniger als 2,5 Gewichtsprozent, vorzugsweise 0,01 bis 1,4 Gewichtsprozent, eines nichtionischen Tensides, wobei eine Menge an nichtionischem Tensid von 0,01 bis 0,7 Gewichtsprozent besonders bevorzugt ist.

Als haarkeratinreduzierender Wirkstoff kommen insbesondere Sulfite oder Mercaptoverbindungen in Betracht, wobei der Gehalt an dem haarkeratinreduzierenden Wirkstoff in der Regel 2 bis 25 Gewichtsprozent beträgt. Beispiele für geeignete Mercaptoverbindungen sind Mercaptocarbonsäuren, wie zum Beispiel Thioglykolsäure und Thiomilchsäure, oder deren Salze. Die Einsatzmenge dieser Mercaptocarbonsäuren beträgt vorzugsweise 2 bis 12 Gewichtsprozent. Es können jedoch auch Sulfite, wie zum Beispiel Alkali- oder Ammoniumsulfite oder -bisulfite, eingesetzt werden. In diesem Fall beträgt die Einsatzmenge im allgemeinen 2 bis 15 Gewichtsprozent. Als haarkeratinreduzierender Wirkstoff können auch Mercaptocarbonsäureester, beispielsweise Thiglykolsäure- oder Thiomilchsäureester verwendet werden, wobei die Einsatzmenge 5 bis 25 Gewichtsprozent beträgt. Ebenfalls können Cystein oder Cysteamin sowie deren Salze, vorzugsweise in einer Menge von 2 bis 25 Gewichtsprozent, verwendet werden.

Das Haarverformungsmittel kann sauer, alkalisch oder neutral eingestellt sein, wobei der pH-Wert im allgemeinen gleich 4 bis 11, vorzugsweise gleich 4 bis 9,5, ist und die Einstellung des pH-Wertes in der Regel mit Ammoniak, Monoethanolamin, Ammoniumcarbonat oder Ammoniumhydrogencarbonat erfolgt.

Als nichtionisches Tensid werden vorzugsweise polyoxyethylierte Fettalkohole, beispielsweise mit 40 Mol Ethylenoxid oxethyliertes Rizinusöl (CTFA-PEG-40 Castor Oil), polyoxyethylierte Nonylphenole, beispielsweise mit 4 oder 10 Mol Ethylenoxid oxethyliertes Nonylphenol (CTFA-Nonoxynol 4 beziehungsweise 10), mit 15 Mol Ethylenoxid oxethyliertes Kokosfettsäureamin (CTFA-PEG-15 Cocoamine), mit 30 Mol Ethylenoxid oxethyliertes hydriertes Lanolin (CTFA-PEG-30 Hydrogenated Lanolin), mit 75 Mol Ethylenoxid oxethyliertes Lanolin (CTFA-PEG-75 Lanolin) und mit 25 Mol Ethylenoxid oxethyliertes hydriertes Rizinusöl (CTFA-PEG-25 Hydrogenated Castor Oil), verwendet, wobei das mit 40 Mol Ethylenoxid oxethylierte Rizinusöl besonders bevorzugt ist.

Die vorstehend genannten CTFA-PEG- beziehungsweise CTFA-Nonoxynol-Verbindungen werden im CTFA International Ingredient Dictionary, 4th Edition (1991), auf den Seiten 383, 385, 397, 400 und 404 beziehungsweise 341 und 343 beschrieben.

Das Haarverformungsmittel kann weiterhin für derartige Mittel übliche Zusatzstoffe, beispielsweise Quell- und Penetrationsstoffe, Verdickungsmittel, Alkohole, wie zum Beispiel Ethanol, Isopropanol oder Glycerin, Lösungsvermittler, Stabilisatoren, Puffersubstanzen, Farbstoffe sowie anionische oder amphotere oberflächenaktive Verbindungen enthalten. Die vorstehend genannten Zusatzstoffe werden in den für solche Zwecke üblichen Mengen verwendet, beispielsweise die Verdickungsmittel in einer Konzentration von 0,1 bis 25 Gewichtsprozent.

Das bei dem erfindungsgemäßen Verfahren verwendete Haarverformungsmittel kann sowohl in Form einer wäßrigen Lösung oder Emulsion als auch in verdickter Form auf wäßriger Basis, insbesondere als Creme, Gel oder Paste vorliegen.

Nach einer für die dauerhafte Haarverformung ausreichenden Einwirkungszeit, welche je nach Haarbeschaffenheit, dem pH-Wert und der Verformungswirksamkeit des Haarverformungsmittels sowie in Abhängigkeit von der Anwendungstemperatur etwa 5 bis 30 (5 bis 20 Minuten mit Wärmeeinwirkung; 20 bis 30 Minuten ohne Wärmeeinwirkung) beträgt, wird das Haar mit Wasser gespült und sodann mit etwa 80 bis 100 Gramm eines Fixiermittels oxidativ nachbehandelt.

Das in dem erfindungsgemäßen Verfahren verwendete Fixiermittel enthält einen oxidierenden Wirkstoff sowie 0,01 bis 6 Gewichtsprozent, vorzugsweise 0,02 bis 1,5 Gewichtsprozent, mindestens eines kationischen Polymers und 0,01 bis 6 Gewichtsprozent, vorzugsweise 0,02 bis 1,5 Gewichtsprozent, mindestens eines kationischen Tensides. Die Gesamtmenge an kationischem Tensid und kationischem Polymer beträgt hierbei vorzugsweise 0,02 bis 7 Gewichtsprozent.

Als oxidierender Wirkstoff kann jede beliebige, bisher in Fixiermitteln verwendete oxidierende Verbindung eingesetzt werden. Beispiele für oxidierende Wirkstoffe sind Alkalibromate, wie zum Beispiel Kalium- und Natriumbromat, oder Wasserstoffperoxid und dessen Additionsverbindungen. Die in dem Fixiermittel enthaltene Menge des oxidierenden Wirkstoffes variiert in Abhängigkeit von der Anwendungsdauer und der Anwendungstemperatur. Normalerweise wird der oxidierende Wirkstoff in einer Menge von etwa 0,5 bis 30 Gewichtsprozent eingesetzt.

Als geeignete kationische Tenside sind Alkyltrimethylammoniumsalze, beispielsweise Lauryltrimethylammoniumchlorid, Tetradecyltrimethylammoniumchlorid, Cetyltrimethylammoniumchlorid oder Stearyltrimethylammoniumchlorid, Cetyldimethylhydroxyethylammoniumdihydrogenphosphat, Cetyldimethylbenzylammoniumchlorid, Alkylpyridiniumsalze, wie zum Beispiel Laurylpyridiniumchlorid oder Cetylpyridiniumchlorid, Cetylbenzyldimethylammoniumchlorid, Decyldimethyloctylammoniumchlorid, Laurinsäurecholinesterchlorid, Betaincetylesterchlorid, Kokosfettsäureamidopropyldimethylacetamidylammoniumchlorid und Dimethyldi-(gehärtete Talgfettsäure)ammoniumchlorid (CTFA-Quaternium-18) zu nennen, wobei Cetyltrimethylammoniumchlorid, Cetylpyridiniumchlorid, Cetylbenzyldimethylammoniumchlorid, Decyldimethyloctylammoniumchlorid und CTFA-Quaternium-18 bevorzugt sind.

Als geeignete kationische Polymere kommen beispielsweise die folgenden Verbindungen oder Mischungen dieser Verbindungen in Betracht: Kationische Cellulosederivate, wie zum Beispiel kationische Celluloseether (beispielsweise CTFA-Polyquaternium-10), mit Methylbromid quaternisierte Polydimethylaminoethylmethacrylate (CTFA-Polyquaternium-9), Polydimethylaminoethylmethacrylat (zu 75 % mit Dimethylsulfat quaternisiert), Polydiallyldimethylammoniumchlorid (CTFA-Polyquaternium-6), Diallyldimethylammoniumchlorid/Hydroxyethylcellulose-Copolymer (CTFA-Polyquaternium-4), beta-Methacryloxyethyl-trimethylammoniummethosulfat-Homopolymer (CTFA-Polyquaternium-14), beta-Methacryloxyethyltrimethylammoniummethosulfat/Acrylamid-Copolymere (CTFA-Polyquaternium-5), Copolymer aus Vinylpyrrolidon und Dimethylaminoethylmethacrylat quaternisiert mit Diethylsulfat (CTFA-Polyquaternium-11), Diallyldimethylammoniumchlorid/Acrylamid-Copolymere (CTFA-Polyquaternium 7), Diallyldimethylammoniumchlorid/ Acrylsäure-Copolymer (CTFA-Polyquaternium-22), Vinylimidazoliummethochlorid/Vinylpyrrolidon-Copolymere (CTFA-Polyquaternium-16) und kationische Chitosanderivate. Unter diesen kationischen Polymeren ist das CTFA-Polyquaternium-11 besonders bevorzugt.

Die vorstehend genannten CTFA-Quaternium- beziehungsweise CTFA-Polyquaternium-Verbindungen werden im CTFA International Cosmetic Ingredient Dictionary, 4th Edition (1991), auf den Seiten 509 beziehungsweise 462 bis 464 beschrieben.

In einer bevorzugten Ausführungsform enthält das Fixiermittel neben dem oxidierenden Wirkstoff, dem kationischen Tensid und dem kationischen Polymer als zusätzlichen Wirkstoff ein wasserlösliches Silikontensid.

Als wasserlösliches Silikontensid werden vorzugsweise Dimethylpolysiloxane mit Polyoxyethylen - und/oder Polyoxypropylenseitenketten (CTFA-Dimethicone Copolyol), die Butyl- oder Methylether des CTFA-Dimethicone Copolyols (CTFA-Dimethicone Copolyol Butyl Ether beziehungsweise CTFA-Dimethicone Copolyol Methyl Ether) sowie der Teilester des CTFA-Dimethicone Copolyols mit Essigsäure (CTFA-Dimethicone Copolyol Acetate) eingesetzt, wobei das CTFA-Dimethicone Copolyol besonders bevorzugt ist.

Die Menge an Silikontensid in dem Fixiermittel beträgt vorzugsweise 0,01 bis 6 Gewichtsprozent.

Die vorstehend genannten CTFA-Dimethicone Copolyole werden im CTFA International Cosmetic Ingredient Dictionary, 4th Edition (1991), auf den Seiten 161 und 162 beschrieben.

Das Fixiermittel kann in Form einer wäßrigen Lösung oder Emulsion sowie in verdickter Form auf wäßriger Basis, insbesondere als Creme, Gel oder Paste vorliegen. Es kann hierbei sowohl gebrauchsfertig als auch in Form eines Konzentrates vorliegen, welches vor der Anwendung mit Wasser, vorzugsweise in einem Verhältnis von 1 zu 1, verdünnt wird.

Der pH-Wert des gebrauchsfertigen Fixiermittels beträgt in Abhängigkeit vom verwendeten oxidierenden Wirkstoff vorzugsweise 1,5 bis 10,5. So beträgt der pH-Wert bei Verwendung von Wasserstoffperoxid 1,5 bis 6, während bei Verwendung von Alkalibromaten ein pH-Wert von 6 bis 10,5 bevorzugt ist.

Selbstverständlich kann das Fixiermittel alle für derartige Mittel üblichen Zusatzstoffe, zum Beispiel nichtionische Netzmittel oder Emulgatoren, Puffersubstanzen, Peroxidstabilisatoren, wie zum Beispiel aromatische Sulfonsäuren, ortho-Phosphorsäure, Poly- oder Pyrophosphorsäuren oder $\alpha$-Bisabolol, Quell- und Penetrationsstoffe, Farbstoffe, Verdickungsmittel, wie zum Beispiel Kaolin, Bentonite, Fettsäuren, höhere Fettalkohole, Cellulosederivate, Stärke, Alginate oder Polyacrylsäure, Farbstoffe, Trübungsmittel, wie zum Beispiel Polyethylenglykolester, Alkohole, wie zum Beispiel Ethanol, Propanol, Isopropanol oder Glycerin, oder Parfümöle enthalten.

Nach einer Einwirkungszeit von etwa 2 bis 15 Minuten, vorzugsweise von 5 bis 10 Minuten, werden die Wickler entfernt und das abgewickelte Haar, falls erforderlich, nochmals mit dem Fixiermittel oxidativ nachbehandelt. Sodann wird das Haar mit Wasser gespült, zur Frisur gelegt und getrocknet.

Das vorstehend beschriebene erfindungsgemäße Verfahren zur dauerhaften Haarverformung ermöglicht eine gleichmäßige Verformung der Haare bei gleichzeitiger hervorragender Konditionierung der Haare, insbesondere bezüglich Griff und Kämmbarkeit der Haare.

Die Erfindung betrifft weiterhin ein 2-Komponenten-Mittel zur Durchführung des vorstehend beschriebenen Verfahrens zur dauerhaften Haarverformung, welches dadurch gekennzeichnet ist, daß die erste Komponente ein Haarverformungsmittel ist, welches einen haarkeratinreduzierenden Wirkstoff sowie weniger als 2,5 Gewichtsprozent, vorzugsweise 0,01 bis 1,4 Gewichtsprozent, eines nichtionischen Tensides enthält, und die zweite Komponente ein Fixiermittel ist, welches einen oxidierenden Wirkstoff sowie 0,01 bis 6 Gewichtsprozent eines kationischen Tensides und 0,01 bis 6 Gewichtsprozent eines kationischen Polymers enthält.

Das in diesem 2-Komponenten-Mittel verwendete Fixiermittel kann in einer besonders bevorzugten Ausführungsform zusätzlich 0,01 bis 6 Gewichtsprozent eines wasserlöslichen Silikontensides enthalten.

Ebenfalls ist es möglich, daß das Fixiermittel in Form eines Konzentrates vorliegt, welches unmittelbar vor der Anwendung mit Wasser, vorzugsweise im Verhältnis 1 zu 1, verdünnt wird.

Das in diesem 2-Komponenten-Mittel verwendete Haarverformungsmittel kann auch aus 2 Teilen bestehen, die unmittelbar vor der Anwendung miteinander vermischt werden. Eine derartige Konfektionierung des Haarverformungsmittels in 2 Teilen ist besonders dann vorteilhaft, wenn als haarkeratinreduzierender Wirkstoff ein Mercaptocarbonsäureester verwendet wird, da bei Mercaptocarbonsäureestern wegen deren geringer Stabilität in wäßrigen Lösungen in der Regel die Esterkomponente erst unmittelbar vor der Anwendung mit der die restlichen Substanzen enthaltenden wäßrigen Phase vermischt wird.

Die nachfolgenden Beispiele sollen den Gegenstand der Erfindung näher erläutern, ohne diesen hierauf zu beschränken.

**Beispiele**

**Beispiel 1**

**Komponente I: Haarverformungsmittel**

| | |
|---|---|
| 12,0 g | Ammoniumthioglykolat (70-%ige wäßrige Lösung) |
| 2,0 g | Ammoniumhydrogencarbonat |
| 0,8 g | Ammoniak (28-%ige wäßrige Lösung) |
| 0,2 g | mit 40 Mol Ethylenoxid oxethyliertes Rizinusöl |
| 0,1 g | Parfümöl |
| 84,9 g | Wasser |
| 100,0 g | |
| Der pH-Wert dieses Haarverformungsmittels beträgt 8,4. | |

**Komponente IIa: Fixiermittel**

| | |
|---|---|
| 7,0 g | Wasserstoffperoxid (35-%ige wäßrige Lösung) |
| 2,0 g | Copolymer aus Vinylpyrrolidon und Dimethylaminoethylmethacrylat, quaternisiert mit Diethylsulfat (20-%ige wäßrige Lösung) (Handelsname: Gafquat® 755 N der GAF Corp.) |
| 0,2 g | Phosphorsäure (85-%ige wäßrige Lösung) |
| 0,1 g | Cetyltrimethylammoniumchlorid |
| 90,7 g | Wasser |
| 100,0 g | |
| Das Fixiermittel besitzt einen pH-Wert von 2,0. | |

**Komponente IIb: Fixiermittel**

| | |
|---|---|
| 7,0 g | Wasserstoffperoxid (35-%ige wäßrige Lösung) |
| 2,0 g | Copolymer aus Vinylpyrrolidon und Dimethylaminoethylmethacrylat, mit Diethylsulfat qaauternisiert (20-%ige wäßrige Lösung) (Handelsname: Gafquat® 755 N der GAF Corp.) |
| 0,5 g | CTFA-Dimethicone-Copolyol (Surfactant 193 der Firma Dow Corning Europa, Brüssel/Belgien) |
| 0,2 g | Phosphorsäure (85-%ige wäßrige Lösung) |
| 0,1 g | Cetyltrimethylammoniumchlorid |
| 90,2 g | Wasser |
| 100,0 g | |
| Der pH-Wert dieses Fixiermittels beträgt 2,0. | |

Mittellanges, vorgeschädigtes, poröses Haar wird mit einem Shampoo gewaschen, frottiert und auf Wickler mit einem Durchmesser von 8 Millimetern gewickelt. Anschließend wird das vorstehend beschriebene Haarverformungsmittel auf dem gewickelten Haar verteilt. Nach einer Einwirkungszeit von 25 Minuten wird das gewickelte Haar mit Wasser gespült und mit einem Handtuch leicht angetrocknet. Anschließend wird das gewickelte Haar mit dem Fixiermittel (entweder Komponente IIa oder Komponente IIb) oxidativ nachbehandelt. Nach einer Einwirkungszeit von 5 Minuten werden die Wickler entfernt und die Haare mit Wasser gespült. Anschließend wird das Haar mit einem Handtuch leicht frottiert, zur Frisur gelegt und getrocknet.

Das so behandelte Haar fühlt sich weich und glatt an und besitzt eine gute Naßkämmbarkeit, wobei das mit dem Fixiermittel der Komponente IIb behandelte Haar eine im Vergleich zu dem mit der Fixierung der Komponente IIa behandelten Haar bessere Konditionierung aufweist.

**Beispiel 2**

**Komponente I: Haarverformungsmittel**

| | |
|---|---|
| 12,0 g | Ammoniumthioglykolat (70-%ige wäßrige Lösung) |
| 2,0 g | Ammoniumhydrogencarbonat |
| 0,8 g | Ammoniak (28-%ige wäßrige Lösung) |
| 0,2 g | mit 40 Mol Ethylenoxid oxethyliertes Rizinusöl |
| 0,1 g | Parfümöl |
| 84,9 g | Wasser |
| 100,0 g | |

Das Haarverformungsmittel besitzt einen pH-Wert von 8,4.

**Komponente II: Fixiermittel**

| | |
|---|---|
| 9,0 g | Natriumbromat |
| 2,0 g | Copolymer aus Vinylpyrrolidon und Dimethylaminoethylmethacrylat, mit Diethylsulfat quaternisiert (20-%ige wäßrige Lösung), (Handelsname: Gafquat® 755 N der GAF Corp.) |
| 0,5 g | CTFA-Dimethicone-Copolyol (Surfactant 193 der Firma Dow Corning Europe, Brüssel/Belgien) |
| 0,1 g | Cetyltrimethylammoniumchlorid |
| 88,4 g | Wasser |
| 100,0 g | |

Das Fixiermittel weist einen pH-Wert von 6,0 auf.

Mittellanges gefärbtes Haar wird mit einem Shampoo gewaschen, frottiert und auf Wickler mit einem Durchmesser von 10 Millimetern gewickelt. Anschließend wird das vorstehend beschriebene Haarverformungsmittel mit einer Auftrageflasche auf die gewickelten Haare aufgetragen. Nach einer Einwirkungszeit von 20 Minuten wird das gewickelte Haar mit lauwarmem Wasser gespült und mit einem Handtuch trockenfrottiert.

Sodann wird das Haar mit 80 g des vorstehenden Fixiermittels oxidativ nachbehandelt. Nach einer Einwirkungszeit von 20 Minuten werden die Wickler entfernt und das abgewickelte Haar mit 40 g des vorstehend beschriebenen Fixiermittels erneut oxidativ nachbehandelt.

Anschließend wird das Haar mit lauwarmem Wasser gespült, mit einem Handtuch frottiert und sodann zur Frisur gelegt und getrocknet.

Das so behandelte Haar weist eine sehr gute Umformung ohne Überkrausung auf und besitzt einen glatten, weichen Griff.

6

**Beispiel 3**

**Komponente I: Haarverformungsmittel**

| Zusammensetzung A: | |
|---|---|
| 7,0 g | Ammoniak (28-%ige wäßrige Lösung) |
| 0,3 g | mit 40 Mol Ethylenoxid oxethyliertes Rizinusöl |
| 0,1 g | Parfümöl |
| 92,6 g | Wasser |
| 100,0 g | |

| Zusammensetzung B: | |
|---|---|
| 80 g | Thioglykolsäuremonoglycerinester |
| 20 g | Glycerin |
| 100 g | |

Vor Gebrauch werden 75 g der Zusammensetzung A und 25 g der Zusammensetzung B miteinander vermischt. Der pH-Wert des so erhaltenen Haarverformungsmittels beträgt 8,3.

Das gewaschene und frottierte Haar wird auf Wickler mit einem Durchmesser von 6 Millimetern gewickelt und sodann mit dem vorstehend beschriebenen Haarverformungsmittel gleichmäßig durchfeuchtet. Anschließend wird das Haar mit einer Plastikhaube abgedeckt und 15 Minuten lang auf 40 Grad Celsius erwärmt. Sodann wird die Abdeckung entfernt, das Haar mit Wasser gespült und mit 100 g eines Fixiermittels gemäß Komponente IIb aus Beispiel 1 oxidativ nachbehandelt. Nach Entfernung der Wickler werden die Haare mit Wasser gespült, zur Frisur gelegt und getrocknet.

Als Ergebnis dieser Behandlung wird eine gleichmäßige, natürlich wirkende Umformung der Haare vom Haaransatz bis zu den Haarspitzen bei gleichzeitiger hervorragender Konditionierung der Haare erhalten.

**Beispiel 4: Vergleichsversuche**

**A. Halbseitenversuch zum Nachweis der verbesserten Haarkonditionierung**

Zu Vergleichszwecken wurde ein 2-Komponenten-Mittel gemäß Beispiel 1 (Komponente I in Verbindung mit Komponente IIb) im Halbseitenversuch mit einem Mittel verglichen, bei dem in der Komponente I der Gehalt an nichtionischem Tensid (mit 40 Mol Ethylenoxid ethoxyliertes Rizinusöl) von 0,2 Gewichtsprozent auf 3 Gewichtsprozent erhöht worden war, während das Fixiermittel (Komponente IIb) unverändert blieb.

Die Haare wurden gewaschen und frottiert und sodann in der Mitte gescheitelt. Während die eine Hälfte mit einem Haarverformungsmittel gemäß Beispiel 1 in der dort beschriebenen Weise behandelt wurde, wurde die andere Hälfte mit einem Haarverformungsmittel gemäß Beispiel 1, bei dem jedoch die Menge an mit 40 Mol Ethylenoxid oxethyliertem Rizinusöl von 0,2 auf 3 Gewichtsprozent erhöht worden war, in der gleichen Weise behandelt. Nach einer Einwirkungszeit von 25 Minuten wurden die Haare mit Wasser gespült und mit einem Fixiermittel gemäß Komponente IIb aus Beispiel 1 fixiert (Einwirkungszeit des Fixiermittels: 5 Minuten), sodann abgewickelt, mit Wasser gespült, zur Frisur gelegt und getrocknet.

Während das nach dem erfindungsgemäßen Verfahren behandelte Haar ein glattes und gepflegtes Aussehen besaß und leicht kämmbar war, wirkte das mit dem nicht-erfindungsgemäßen Verfahren behandelte Haar unangenehm stumpf und besaß eine schlechtere Kämmbarkeit.

**B. Halbseitenversuch zum Nachweis der verbesserten Verträglichkeit mit kationischen Haarkurmitteln**

Zunächst wurden die Haare in der in Beispiel 1 (Komponente I in Verbindung mit Komponente IIb) beschriebenen Weise umgeformt. Im Anschluß an die Haarverformungsbehandlung wurde das feuchte Haar in der Mitte gescheitelt und die eine Hälfte der Haare mit einem kationischen Haarkurmittel der folgenden Zusammensetzung behandelt, während die andere Hälfte unbehandelt blieb.

**Haarkurmittel:**

| | |
|---|---|
| 4,00 g | Cetylalkohol |
| 3,00 g | Cetyltrimethylammoniumchlorid |
| 0,50 g | Vaseline (Petrolatum) |
| 0,50 g | selbstemulgierendes Glycerinmonostearat |
| 0,05 g | mit 25 Mol Ethylenoxid oxethylierter Cetylalkohol |
| 91,95 g | Wasser |
| 100,00 g | |

Nach einer Einwirkungszeit von 2 Minuten wurde das Haarkurmittel mit lauwarmem Wasser ausgespült und das Haar mit einem Handtuch frottiert.

Durch die Behandlung mit dem Haarkurmittel wurde der Griff des Haares sowie seine Naßkämmbarkeit im Vergleich zu dem unbehandelten Haar geringfügig verbessert.

Dieser Vergleichsversuch zeigt, daß bei dem erfindungsgemäßen Verfahren nach der Anwendung von kationischen Haarkurmitteln keinerlei Probleme auftreten und der bei üblichen Haarverformungsverfahren häufig beobachtete "Antieffekt", das heißt eine Verschlechterung der Haarstruktur durch eine Nachbehandlung mit kationischen Haarkurmitteln, nicht auftritt.

Alle in der vorliegenden Anmeldung enthaltenen Prozentangaben stellen, soweit nicht anders angegeben, Gewichtsprozente dar.

**Patentansprüche**

1. Verfahren zur dauerhaften Haarverformung, bei dem man das Haar bevor und/oder nachdem man es in die gewünschte Form bringt mit einem Haarverformungsmittel auf der Basis eines haarkeratinreduzierenden Wirkstoffes behandelt, mit Wasser spült, anschließend mit einem Fixiermittel auf der Basis eines oxidierenden Wirkstoffes oxidativ nachbehandelt, mit Wasser spült, zur Frisur legt und sodann trocknet, dadurch gekennzeichnet, daß ein Haarverformungsmittel, welches weniger als 2,5 Gewichtsprozent eines nichtionischen Tensides enthält, und ein Fixiermittel, welches 0,01 bis 6 Gewichtsprozent eines kationischen Tensides und 0,01 bis 6 Gewichtsprozent eines kationischen Polymers enthält, verwendet wird.

2. Verfahren nach Anspruch 1, dadurch gekennzeichnet, daß man das Haarverformungsmittel 5 bis 30 Minuten einwirken läßt.

3. Verfahren nach Anspruch 1, dadurch gekennzeichnet, daß man das Fixiermittel 2 bis 15 Minuten einwirken läßt.

4. Verfahren nach einem der Ansprüche 1 bis 3, dadurch gekennzeichnet, daß das nichtionische Tensid ausgewählt ist aus polyoxethylierten Fettalkoholen, polyoxethylierten Nonylphenolen, CTFA-PEG-15 Cocoamine, CTFA-PEG-30 Hydrogenated Lanolin, CTFA-PEG-75 Lanolin und CTFA-PEG-25 Hydrogenated Castor Oil.

5. Verfahren nach einem der Ansprüche 1 bis 4, dadurch gekennzeichnet, daß das kationische Tensid ausgewählt ist aus Lauryltrimethylammoniumchlorid, Tetradecyltrimethylammoniumchlorid, Cetyltrimethylammoniumchlorid, Stearyltrimethylammoniumchlorid, Cetyldimethylhydroxyethylammoniumdihydrogenphosphat, Cetyldimethylbenzylammoniumchlorid, Laurylpyridiniumchlorid, Cetylpyridiniumchlorid, Cetylbenzyldimethylammoniumchlorid, Decyldimethyloctylammoniumchlorid, Laurinsäurecholinesterchlorid, Betaincetylesterchlorid, Kokosfettsäureamidopropyldimethylacetamidylammoniumchlorid und CTFA-Quaternium-18.

6. Verfahren nach einem der Ansprüche 1 bis 5, dadurch gekennzeichnet, daß das kationische Polymer ausgewählt ist aus CTFA-Polyquaternium-10, CTFA-Polyquaternium-9, Polydimethylaminoethylmethacrylat (zu 75 % mit Dimethylsulfat quaternisiert), CTFA-Polyquaternium-6, CTFA-Polyquaternium-4, CTFA-Polyquaternium-14, CTFA-Polyquaternium-5, CTFA-Polyquaternium-11, CTFA-Polyquaternium-7, CTFA-Polyquaternium-22, CTFA-Polyquaternium-16 und kationischen Chitosanderivaten.

7. Verfahren nach einem der Ansprüche 1 bis 6, dadurch gekennzeichnet, daß das Fixiermittel zusätzlich ein wasserlösliches Silikontensid enthält.

8. Verfahren nach Anspruch 7, dadurch gekennzeichnet, daß das Silikontensid ausgewählt ist aus CTFA-Dimethicone Copolyol, CTFA-Dimethicone Copolyol Butyl Ether, CTFA-Dimethicone Copolyol Methyl Ether und CTFA-Dimethicone Copolyol Acetate.

9. Verfahren nach Anspruch 7 oder 8, dadurch gekennzeichnet, daß das Silikontensid in einer Menge von 0,01 bis 6 Gewichtsprozent enthalten ist.

10. 2-Komponenten-Mittel zur Durchführung des Verfahrens zur dauerhaften Haarverformung nach einem der Ansprüche 1 bis 9, dadurch gekennzeichnet, daß die erste Komponente ein Haarverformungsmittel ist, welches einen haarkeratinreduzierenden Wirkstoff sowie weniger als 2,5 Gewichtsprozent eines nichtionischen Tensides enthält, und die zweite Komponente ein Fixiermittel ist, welches einen oxidierenden Wirkstoff sowie 0,01 bis 6 Gewichtsprozent eines kationischen Tensides und 0,01 bis 6 Gewichtsprozent eines kationischen Polymers enthält.

11. 2-Komponenten-Mittel gemäß Anspruch 10, dadurch gekennzeichnet, daß das Fixiermittel der Komponente 2 zusätzlich 0,01 bis 6 Gewichtsprozent eines wasserlöslichen Silikontensides enthält.

12. 2-Komponenten-Mittel gemäß Anspruch 10, dadurch gekennzeichnet, daß das Haarverformungsmittel der Komponente 1 aus 2 Teilen besteht, die unmittelbar vor der Anwendung miteinander vermischt werden.